# EUROPEAN PATENT APPLICATION

(11) **EP 4 782 825 A1**
(43) Date of publication of application: **29.07.2026**
(21) Application number: 24915752.0
(22) Date of filing: 24.01.2024
(51) Int. Cl.: G01N 27/12, G01N 33/00, G06N 3/08

(54) **GAS SENSING DEVICE**

(30) Priority: 02.01.2024 KR 20240000545
(71) Applicant: LG Electronics Inc., Yeongdeungpo-gu Seoul 07336 (KR)
(72) Inventor: CHO, Young Hee, Seoul 08592 (KR); RYU, Dae Ryuc, Seoul 08592 (KR); KIM, Moosub, Seoul 08592 (KR); MOON, Muhyun, Seoul 08592 (KR); NOH, Jinhee, Seoul 08592 (KR)
(74) Representative: Vossius & Partner Patentanwälte Rechtsanwälte mbB
(86) International application number: PCT/KR2024/001142
(87) International publication number: WO 2025/146860

(57) **Abstract**

The present invention relates to a gas sensing device. In the present invention, a processor (20), a first gas sensor (30), and a second gas sensor (32) are installed on a substrate (10). The first gas sensor (30) and the second gas sensor (32) are positioned between the two ends of the processor (20) and the corresponding edges of the substrate (10), respectively. A temperature/humidity sensor (34) is installed on the substrate (10), and a through channel (12) is formed through the substrate (10) between the temperature/humidity sensor (34) and the processor (20). The first gas sensor (30) and the second gas sensor (32) detect the types and concentrations of a plurality of gases, respectively, and the processor (20) detects a mixed gas and analyzes the types and concentrations of each gas of the detected mixed gas using a deep learning model. At this time, the learning model uses a first-stage classification model and a second-stage regression model.

## Description

### Technical Field

The present disclosure relates to a gas sensing device for sensing multiple types of harmful gases in real time.

### Background Art

As air pollution has intensified, interest in air quality contamination has increased, and accordingly, demands for management and maintenance of indoor air quality are increasing. Not only in industrial sites but also in everyday living environments including homes, emissions of various harmful gases are increasing, and types thereof are also becoming more diverse.

Accordingly, technologies for monitoring indoor environmental information in real time have been developed, and as a result, demand for gas sensors for detecting various harmful gases that cause phenomena such as sick building syndrome has recently increased significantly.

In general, a gas sensor detects harmful gases in air and detects components and concentrations of the corresponding gases. Gas sensors may be classified into various types of gas sensors, such as semiconductor-type, catalytic-type, and optical-type gas sensors, according to a measurement method. Among these, the semiconductor-type gas sensor has been actively researched due to the use of metal oxides that enable a simple manufacturing process at a low cost.

As a conventional technology, a technique for detecting harmful gases by using a gas sensor has been proposed. For example, as disclosed in Korean Patent Application Publication No. 10-2021-0104390, a contamination state is detected by outputting a signal corresponding to ionic electrical conductivity of an electrolyte of a gas sensor. However, since the ionic electrical conductivity varies depending on temperature, an output value is increased or decreased whenever the temperature changes, and when the output value is corrected, and the corrected value is equal to or greater than a threshold value, it is determined that a toxic gas has been detected. However, although a plurality of gas sensors are provided for detecting multiple types of gases, only a current value of each of the sensors is measured, and thus an actual concentration value of a gas cannot be obtained, and since an electrolyte is used, durability is reduced, and electrical conductivity varies depending on temperature, thereby increasing an error rate. In addition, since a gas sensor having a large size is used, there is a problem in that a device size increases in proportion to the number of detected gases in order to detect multiple types of gases.

As another example, Korean Patent No. 10-1674048 discloses a device that includes a function of detecting harmful chemical substances and a network-based communication function, thereby enabling rapid transmission of information when an accident occurs. The device includes a preprocessing part for concentrating harmful gases and maintaining predetermined temperature and humidity, an environmental sensor part for collecting external environmental information, a communication part for communication with an external device, a sensor array part for detecting multiple types of harmful gases, a display, an input part, and a control part, and it is disclosed that most of these components are installed within a single casing.

However, in this case, since a heater for maintaining temperature and discharging adsorbed gas is provided, a barrier is required to prevent heat generated by the heater from affecting other components, thereby increasing the size of the gas measurement device. In addition, use of the heater causes a problem in that power consumption is increased, and since the adsorbed gas must be discharged, a sensor response is not performed in real time. Further, provision of the harmful gas concentration part implies that a sensor is capable of detection only after concentrating gas, and thus there is a problem in that detection at a low concentration and real-time detection are difficult.

As another example, Korean Patent Application Publication No. 10-2022-0142030 discloses a method in which VOC gases are detected by using a gas sensor and VOC gases in a transient state are rapidly classified by using a recurrent neural network.

However, it is necessary to predict not only types but also concentrations of indoor harmful gases in real time, to correct sensitivity values that vary depending on temperature and humidity so as to obtain a constant output value, and to miniaturize the overall size of a device so as to minimize an installation space required.

### Disclosure

### Technical Problem

The present disclosure provides a gas sensing device having a miniaturized overall device size.

The present disclosure provides a gas sensing device that detects gas by using a gas sensor and accurately provides a type and a concentration of the gas.

The present disclosure is intended to enable real-time detection of a type and a concentration of gas while an overall device is miniaturized.

The present disclosure provides a gas concentration and a gas type by training a deep learning model on the basis of pre-prepared training data and analyzing a type and a concentration of gas.

The present disclosure is intended to correct sensitivity values that vary depending on temperature and humidity so as to consistently output a constant output value.

### Technical Solution

Gas sensors used in a gas sensing device of the present disclosure may detect types and concentrations of multiple gases in real time.

In the present disclosure, training data on sensing sensitivity of a sample gas may be obtained, a deep learning model may be trained by using the training data, and the deep learning model may be further trained on the basis of sensing sensitivity of a detected target gas to analyze a type and a concentration of the target gas.

In the present disclosure, with respect to a processor that analyzes a type and a concentration of a target gas, a first gas sensor, a second gas sensor, and a temperature/humidity sensor may be installed at predetermined angular intervals at opposite ends of the processor.

The first gas sensor, the second gas sensor, and the temperature/humidity sensor may be respectively disposed between edges of the processor and corresponding edges of a substrate.

A gas sensing device of the present disclosure includes: a substrate, a processor installed on the substrate and configured to analyze detected data, and a first gas sensor configured to detect different types of target gases and a second gas sensor configured to detect different types of target gases, the first gas sensor and the second gas sensor being installed with the processor interposed therebetween, wherein the first gas sensor is positioned between a first edge of the processor and a corresponding edge of the substrate, and the second gas sensor is positioned between a second edge of the processor and a corresponding edge of the substrate.

The processor may analyze a type and a concentration of each of the detected target gases.

The processor may acquire training data for sensing sensitivity of sample gases, train a deep learning model by using the training data, train the deep learning model by using sensing sensitivity of the detected target gases, and analyze a type and a concentration of each of the target gases, wherein the deep learning model may include a first-stage classification model and a second-stage regression model.

The classification model may be a fully connected neural network (FCN), and the regression model may be multiple linear regression (MLR).

The FCN may receive the sensing sensitivity of the detected target gases and output the type of each of the target gases, and the MLR may receive the type of each of the target gases output from the FCN and the sensing sensitivity of the detected target gases and output the concentration of the target gas.

The processor may acquire sensing sensitivity of the plurality of sample gases, generate distribution data of the sensing sensitivity, generate normal distribution data on the basis of an average and a standard deviation of the distribution data, and acquire the normal distribution data as the training data.

One of the first gas sensor and the second gas sensor may detect ethanol (C₂H₆O), formaldehyde (HCHO), toluene (C₇H₈), and hydrogen (H₂).

A temperature/humidity sensor may be installed between one edge of the substrate and an edge of the processor.

A through-channel may be formed to penetrate the substrate between the temperature/humidity sensor and the processor.

The through-channel may be formed to surround the temperature/humidity sensor except for a partial section thereof.

The temperature/humidity sensor, the first gas sensor, and the second gas sensor may be respectively disposed around the processor at 90° intervals between corresponding edges of the processor and the substrate.

A connector for signal connection with an external device may be installed at one edge of the substrate, wherein the connector may be positioned offset toward one side along the edge.

An ESD diode for electrostatic discharge may be used on the substrate.

The substrate may include a cover configured to shield, from an external environment, a surface on which the processor is installed, wherein the cover may include a processor shielding part formed at a position corresponding to the position of the processor, wherein a first sensor window, a second sensor window, and a third sensor window may be respectively formed between corresponding edges of the processor shielding part and the cover.

A partition grid may be formed in the third sensor window.

### Advantageous Effects

The gas sensing device according to the present disclosure may have at least one of the following effects.

According to the present disclosure, the gas sensing device that is miniaturized while detecting types and concentrations of multiple harmful gases in real time may be provided. This may be achieved by arranging multiple MEMS gas sensors with other components installed on the substrate interposed therebetween to secure a required distance between the gas sensors, and by forming the through-channel in the substrate to block thermal influence from surrounding components on the temperature/humidity sensor. Accordingly, distances between components mounted on the substrate may be minimized, thereby enabling miniaturization of the overall device.

According to the present disclosure, the deep learning model may be applied, and accordingly, types and concentrations of detected gases may be provided in real time.

According to the present disclosure, by applying a deep learning model, components and concentrations of respective gases in a mixed gas mixed at various ratios can be analyzed.

In addition, according to the present disclosure, sensitivity values that vary depending on temperature and humidity may be corrected. To this end, sensor sensitivity may be corrected by using an absolute humidity value. Through this, sensitivity values corresponding to differences in absolute humidity may be made similar and applied to a deep learning algorithm, thereby significantly reducing an error rate.

### Description of Drawings

FIG. 1 is a block diagram illustrating a configuration of a preferred embodiment of a gas sensing device of the present disclosure.
FIG. 2 is a plan view illustrating main parts of an embodiment of the present disclosure.
FIG. 3 is a plan view illustrating a cover that shields a substrate shown in FIG. 2 in an embodiment of the present disclosure.
FIG. 4 is a diagram illustrating a configuration of a gas sensor constituting the gas sensing device according to an embodiment of the present disclosure.
FIG. 5 is a diagram illustrating a configuration of a device that uses a sample gas to acquire training data used in an embodiment of the present disclosure.
FIGS. 6a to 6c are diagrams illustrating a process of obtaining normal distribution data by using training data of a sample gas according to the present embodiment.
FIG. 7 is an example diagram illustrating a process for training a deep learning model used in an embodiment of the present disclosure.
FIG. 8 is an example diagram illustrating a deep learning model used in an embodiment of the present disclosure.
FIG. 9 is a flowchart illustrating a gas analysis method used in an embodiment of the present disclosure.
FIG. 10 is an example diagram illustrating a process for a gas analysis experiment used in an embodiment of the present disclosure.
FIG. 11 is an experimental result diagram illustrating discriminative capability for each gas according to the experiment of FIG. 10.
FIG. 12 is an experimental result diagram illustrating a concentration error rate for each gas according to the experiment of FIG. 10.

### Best Mode

Hereinafter, some embodiments of the present disclosure will be described in detail with reference to exemplary drawings. When adding reference numerals to components in each drawing, it should be noted that identical components are given the same reference numerals as much as possible even if they are shown in different drawings. In addition, when describing the embodiments of the present disclosure, if a detailed description of the relevant known configuration or function is judged to hinder understanding of the embodiments of the present disclosure, detailed descriptions thereof will be omitted.

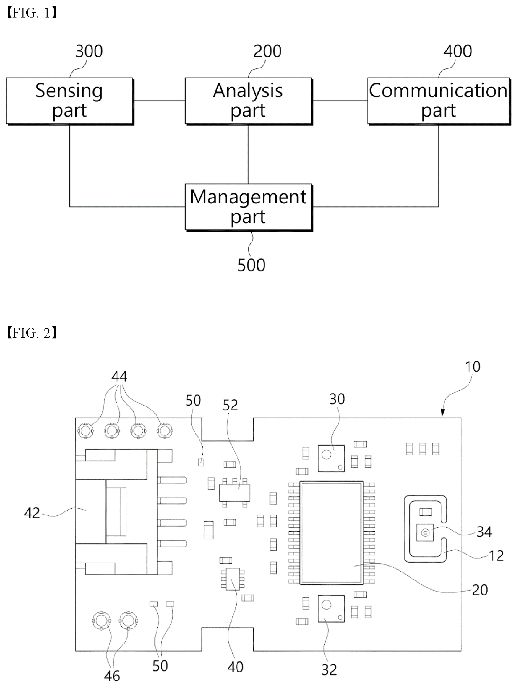

FIG. 1 is a block diagram illustrating a configuration of a gas sensing device according to an embodiment of the present disclosure. The gas sensing device according to the embodiment of the present disclosure may include an analysis part (200), a sensing part (300), a communication part (400), and a management part (500).

The analysis part (200) may be a part for analyzing detected gas-related information and may process a deep learning algorithm. The analysis part (200) may include a processor (20) to be described below. The analysis part (200) may analyze a type and a concentration of gas by using sensing sensitivity of gas detected by the sensing part (300) to be described below. The analysis part (200) may train a deep learning model by using pre-prepared training data for a sample gas, and further train the deep learning model on the basis of sensing sensitivity of a target gas to be analyzed so as to analyze types and concentrations of individual gases contained in the target gas. Accordingly, the analysis part (200) may include a processor or a microcontroller (MICOM) capable of executing software or a program for training the deep learning model and analyzing types and concentrations of gases, or may include a personal computer (PC) equipped with such a processor or microcontroller.

The sensing part (300) may include a first gas sensor (30), a second gas sensor (32), and a temperature/humidity sensor (34) to be described below. The sensing part (300) may detect information on a gas to be detected as well as temperature and humidity around the device. Here, each of the first gas sensor (30) and the second gas sensor (32) may sense different types of mixed gases. That is, the first gas sensor (30) may sense various types of gases and the second gas sensor (32) may also sense various types of gases. Of course, types of gases sensed by the first gas sensor (30) and the second gas sensor (32) may be different from each other.

The communication part (400) may be configured for communication between the device of the present disclosure and an external device. For example, the communication part (400) may include a level shifter (40), a connector (42), and first and second external connection pins (44, 46).

The management part (500) may include an ESD diode (50) and a regulator (42).

Components constituting an embodiment of the present disclosure may be mainly installed on a substrate (10). The substrate (10) may be a general printed circuit board. With reference to the drawing, the printed circuit board may have a dimension of approximately 19mm to 24mm in a vertical direction and a dimension of approximately 33mm to 40mm in a horizontal direction.

Circuit patterns (not shown) may be formed inside the substrate (10), and opposite surfaces thereof may be formed as surface layers made of an insulating material. Portions of the circuit patterns that are exposed may serve as electrodes, and various components may be mounted on the electrodes so as to be electrically connected thereto.

A through-channel (12) may be formed to penetrate the substrate (10). The through-channel (12) may be formed along a location where the circuit patterns are not formed. In the illustrated embodiment, the through-channel (12) may have a C shape. The temperature/humidity sensor (34) to be described below may be installed in a region surrounded by the through-channel (12). The through-channel (12) may serve to block transfer of heat generated from components mounted on the substrate (10). That is, the through-channel (12) may serve to prevent heat generated from surrounding components from being transferred to the temperature/humidity sensor (34), thereby preventing operation of the temperature/humidity sensor (34) from being affected.

The processor (20) may be mounted on the substrate (10). The processor (20) may execute software or a program for training a deep learning model to analyze types and concentrations of gases. In addition, the processor (20) may control the process of storing various data or transmitting the data to an external device. The processor (20) may be disposed such that a center thereof is positioned on a vertical centerline with reference to the drawing. That is, distances from an upper end and a lower end (with reference to the drawing) of the processor (20) to corresponding edges of the substrate (10) may be equal. The processor (20) may include multiple leads (not shown) soldered to the electrodes of the substrate (10), thereby electrically connecting the processor (20) to the circuit patterns.

The substrate (10) may include the first gas sensor (30) and the second gas sensor (32). For example, the first gas sensor (30) may be disposed at an upper side of the processor (20) (with reference to the drawing), and the second gas sensor (32) may be disposed at a lower side of the processor (20) (with reference to the drawing). That is, the first gas sensor (30) and the second gas sensor (32) may be positioned with the processor (20) interposed therebetween. That is, the first gas sensor (30) and the second gas sensor (32) may be respectively disposed in regions between the opposite longitudinal ends of the processor (20) and the corresponding edges of the substrate (10). Detailed configurations of the first and second gas sensors (30, 32) will be described below.

The temperature/humidity sensor (34) may be installed in the region surrounded by the through-channel (12). A location at which the temperature/humidity sensor (34) is installed may be positioned between an edge of the processor (20) on which the leads are provided and one edge of the substrate (10). Accordingly, the first gas sensor (30), the temperature/humidity sensor (34), and the second gas sensor (32) may be disposed around the processor (20) at approximately 90° intervals between corresponding edges of the processor (20) and the substrate (10). The temperature/humidity sensor (34) may detect ambient temperature and humidity and provide the detected temperature and humidity to the processor (20).

With respect to the processor (20), the level shifter (40) may be disposed on a side opposite to the temperature/humidity sensor (34). The level shifter (40) may be provided for communication with a product in which the present device is used and may be for I²C communication.

The connector (42) may be positioned on one side of the substrate (10) for signal connection between the substrate (10) and an external device (a product in which the present device is used). The connector (42) may be electrically connected to the circuit patterns of the substrate (10), and perform signal connection by being coupled with a separate external connector (not shown). In the illustrated embodiment, the connector (42) may be positioned at one edge of the substrate (10). The connector (42) may be disposed at an edge opposite to a side on which the temperature/humidity sensor (34) is installed.

As can be seen in FIG. 2, the connector (42) may be installed offset toward one side along one edge of the substrate (10). In FIG. 2, the connector (42) is installed at a position relatively biased toward an upper side from a left edge with reference to the drawing. This may serve as a positional reference when the substrate (10) is mounted to a product.

On opposite sides in a width direction of the connector (42), the first external connection pin (44) and the second external connection pin (46) may be provided. The first external connection pin (44) may include a set of four pins, and may be used to download a program used by the processor (20). The second external connection pin (46) may include a set of two pins and may be used to check sensor information.

The substrate (10) may include the ESD diode (50) (50). The ESD diode (50) may include multiple ESD diodes (50). In the illustrated embodiment, three ESD diodes (50) are provided. The ESD diodes (50) may be provided for protection against electrostatic discharge. The ESD diodes (50) may serve to prevent sensors from being impacted by electrostatic discharge generated by contact of an operator during an assembly operation, thereby preventing malfunction.

The substrate (10) may further include a regulator (52). The regulator (52) may convert a voltage supplied from an external source into a voltage for driving the sensors (30, 32, 34). For example, when a voltage used by a product is 5V and a voltage used by the sensors (30, 32, 34) is 3.3V, the regulator (52) may serve to step down the externally supplied voltage of 5V to 3.3V.

Meanwhile, in an embodiment of the present disclosure, a cover (60) may be used. The cover (60) may serve to shield, from an external environment, portions of the substrate (10) on which various components including the processor (20) are installed. The cover (60) may also serve to prevent electrostatic discharge.

The cover (60) may be injection-molded of an insulating material. The cover (60) may include a cover body (61) constituting a framework thereof, the cover body (61) having an edge shape corresponding to a surface shape of one side of the substrate (10). The cover body (61) may include a processor shielding part (62). The processor shielding part (62) may be disposed at a position corresponding to the position of the processor (20) on the substrate (10). The processor shielding part (62) may protrude relative to other portions of the cover body (61). This is designed in consideration of a degree to which the processor (20) protrudes from the substrate (10).

The processor shielding part (62) may include multiple through-holes (62') formed therein. The through-holes (62') may serve to allow heat generated by the processor (20) to be discharged to the outside. The number and sizes of the through-holes (62') may be determined such that the processor shielding part (62) maintains sufficient rigidity to protect the processor (20) while allowing smooth heat dissipation.

Adjacent to the processor shielding part (62), a first sensor window (63) and a second sensor window (63') may be provided. The first and second sensor windows (63, 63') allow the first gas sensor (30) and the second gas sensor (32) to be exposed to the outside so as to facilitate gas detection. To this end, the first sensor window (63) and the second sensor window (63') may be respectively formed at positions corresponding to the first gas sensor (30) and the second gas sensor (32) on the substrate (10). In the present embodiment, each of the first sensor window (63) and the second sensor window (63') may have a rectangular shape. However, the shape is not limited thereto, as long as the first gas sensor (30) and the second gas sensor (32) can be accurately exposed to the outside.

The cover (60) may include a third sensor window (64). The third sensor window (64) may be disposed at a position corresponding to the temperature/humidity sensor (34) on the substrate (10). The third sensor window (64) may be in communication with the outside so that the temperature/humidity sensor (34) may accurately detect temperature and humidity. A partition grid (64') may be formed in the third sensor window (64). The partition grid (64') may allow smooth detection of temperature and humidity by the temperature/humidity sensor (34) through the third sensor window (64) while protecting the temperature/humidity sensor (34).

A substrate hooking lever (66) may be provided at one edge of the cover (60) for coupling the cover (60) to the substrate (10). The substrate hooking lever (66) may include two substrate hooking levers provided side by side. In the present embodiment, the substrate hooking levers (66) may be formed at one edge of the cover body (61) adjacent to the third sensor window (64). Edges orthogonal to opposite ends of the edge on which the substrate hooking levers (66) are formed may be inserted, together with edges of the substrate (10), into a specific structure of a product so as to be hooked thereto.

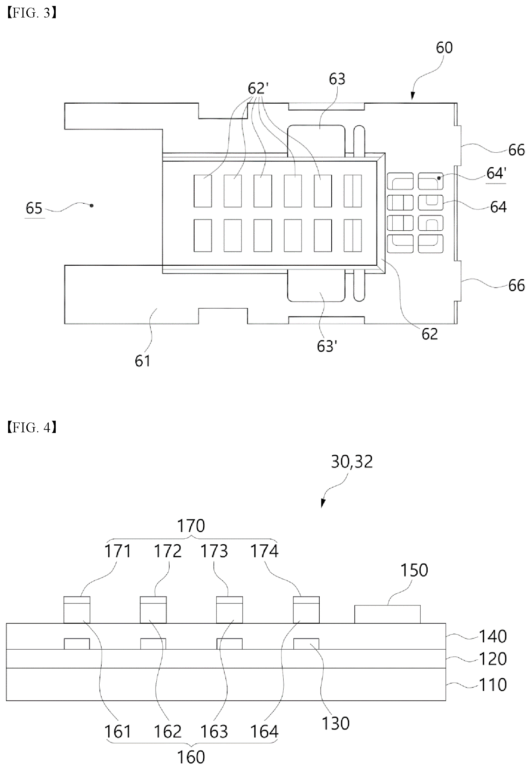

According to FIG. 4, the first and second gas sensors (30, 32) may include a sensor substrate (110). The sensor substrate (110) may be a silicon substrate used in a general semiconductor process, or a ceramic substrate made of Al₂O₃, ZrO₂, MgO, which have favorable characteristics at high temperatures.

A first insulating layer (120) may be formed on an upper surface of the sensor substrate (110). The first insulating layer (120) may be composed of a single layer or multiple layers of a silicon oxide film or a silicon nitride film. A heater (130) may be formed on an upper surface of the first insulating layer (120).

The heater (130) may serve to increase an ambient temperature in order to improve gas sensing characteristics. The heater (130) may be made of a metal such as Pt, Pd, W, or Au, or a conductive metal oxide, and may be formed in various patterns composed of lines. A second insulating layer (140) may be formed on an upper surface of the heater (130). The second insulating layer (140) may be composed of a single layer or multiple layers of a silicon oxide film or a silicon nitride film.

A reference electrode (150) and a plurality of sensing electrodes (160) may be formed on an upper surface of the second insulating layer (140). No sensing film may be formed on an upper surface of the reference electrode (150), whereas sensing films (170) made of different sensing materials may be respectively formed on upper surfaces of the sensing electrodes (160).

In the present disclosure, the sensing electrodes (160) may include four sensing electrodes (161-164). Of course, it is apparent that the number of sensing electrodes (160) may be varied. The sensing films (170) may be made of a sensing material that reacts with gas and may cause a change in electrical characteristics of the sensing electrodes (160) upon reaction with gas.

Since no sensing film is formed on the reference electrode (150), the reference electrode (150) may not react with gas. Electrical connection means for transmitting a gas sensing signal to the analysis part (200) may be provided at opposite ends of the reference electrode (150) and the sensing electrodes (160). The electrical connection means may be soldered to the substrate.

The reference electrode (150) and the sensing electrodes (160) may be made of a metal such as Pt, Pd, W, or Au. The sensing electrodes (160) may be disposed on an upper surface of the heater (130). Accordingly, the sensing electrodes (160) may be heated by heat provided from the heater (130). By appropriately controlling the heat provided from the heater (130), gas sensing sensitivity at the sensing electrodes (160) may be controlled. As illustrated in the drawing, the second insulating layer (140) may be disposed between the heater (130) and the electrodes (150, 160) to electrically insulate the heater (130) from the electrodes (150, 160). The heater (130) may also be connected to an external circuit via heater electrode pads (not shown) and bonding wires (not shown). Meanwhile, in another embodiment, the heater (130) may be disposed at a position of a lower surface of the sensor substrate (110), the position corresponding to the sensing electrodes (160).

The first gas sensor (30) and the second gas sensor (32) used in the device of the present disclosure may detect gases such as ethanol (C₂H₆O), hydrogen (H₂), toluene (C₇H₈), and formaldehyde (HCHO). That is, a first sensing film (171) formed on a first sensing electrode (161) may react with ethanol, and a second sensing film (172) formed on a second sensing electrode (162) may react with hydrogen. A third sensing film (173) formed on a third sensing electrode (163) may react with toluene, and a fourth sensing film (174) formed on a fourth sensing electrode (164) may react with hydrogen.

As the first to fourth sensing films (171-174) react with respective gases, resistance changes of the first to fourth sensing electrodes (161-164) may be transmitted to the analysis part (200) as sensing sensitivity signals through bonding wires. In another embodiment, in order to analyze gases other than the above gases, sensing films made of sensing materials that react with the other gases may be formed on upper surfaces of the sensing electrodes (160).

A resistance of the reference electrode (150) may also be transmitted to the analysis part (200) as a reference signal. The analysis part (200) may analyze a type and a concentration of a target gas to be analyzed by using a magnitude of sensing sensitivity of each of the sensing electrodes (160) relative to the reference signal of the reference electrode (150).

Meanwhile, a temperature sensor (not shown) for measuring a temperature of the heater (130) may be additionally provided. The temperature sensor may be a thermistor.

In the first gas sensor (30) and the second gas sensor (32) with the above configuration, oxidation occurs upon reaction with gas in the sensing films (170) formed on surfaces of the sensing electrodes (160), whereby resistance may be reduced due to movement of electrons within the sensing electrodes (160). The analysis part (200) may detect a target gas on the basis of changes in resistance of each of the sensing electrodes (170).

The number of channels of the sensing electrodes (160) and sensing materials of the sensing films (170) may be determined according to a type of a target gas to be detected. In order to detect different types of target gases, different sensing materials may be used.

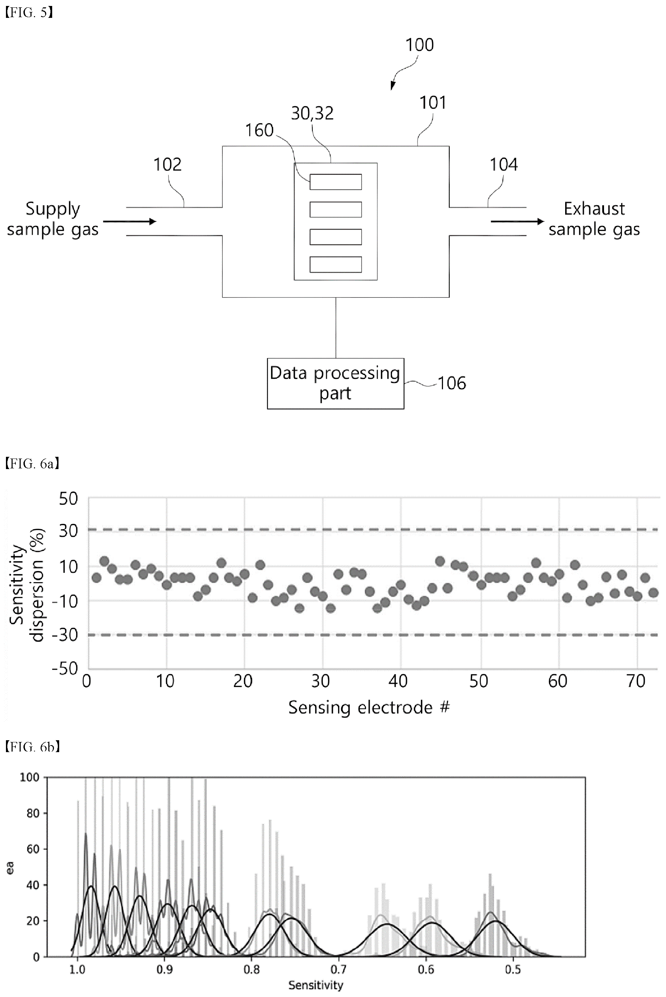

FIG. 5 is a configuration diagram of a device for acquiring training data by using a sample gas to be used in an embodiment of the present disclosure.

Referring to FIG. 5, a training data acquisition device (100) of the present disclosure may include a gas chamber (101). The gas sensors (30, 32) may be disposed in an internal space of the gas chamber (101).

A supply pipe (102) through which a sample gas is supplied from the outside may be formed at one side of the gas chamber (101), and an exhaust pipe (103) through which the sample gas is discharged to the outside may be formed at an opposite side thereof.

While the sample gas supplied into the internal space through the supply pipe (102) is exhausted through the exhaust pipe (103), the gas sensors (30, 32) may detect the sample gas.

In an embodiment of the present disclosure, the gas sensors (30, 32) may include sensing electrodes having four channels, with the sensing electrodes configured to detect ethanol (C₂H₆O), hydrogen (H₂), toluene (C7H8), and formaldehyde (HCHO), respectively.

The gas sensors (30, 32) may be electrically connected to a data processing part (106). The data processing part (106) may receive sensing sensitivity from the gas sensors (30, 32) and may acquire training data for a deep learning model on the basis of the sensing sensitivity for each sample gas.

FIGS. 6a to 6c are diagrams illustrating a process of constructing a training data DB of sample gas according to an embodiment of the present disclosure.

In the present disclosure, for constructing the training data DB, for example, 72 gas sensors (30, 32) may be disposed in the internal space of the gas chamber (101), and sample gases may be supplied into the internal space. The sample gases may be, for example, ethanol, hydrogen, toluene, and formaldehyde, and sensing electrodes (170) of four channels configured to respectively detect these sample gases may be disposed in the gas sensors (100).

In the data processing part (106), training data for a deep learning model may be acquired on the basis of sensing sensitivity for each sensing electrode of the 72 gas sensors (30, 32) so as to construct a database (DB).

Hereinafter, a DB construction process will be described in detail with reference to FIGS. 6a to 6c.

In FIG. 6a, sensing sensitivity of a first gas detected by 72 first sensing electrodes (161) is illustrated as an example. That is, it is sensing sensitivity of the first gas detected by the 72 first sensing electrodes (161).

The sensing sensitivity data of the first gas are converted into distribution data by sensing sensitivity for the first gas as shown in FIG. 6b.

In this case, the distribution data by sensing sensitivity in FIG. 6b do not show distribution data of all sensing sensitivity. That is, when such distribution data are used as training data, an error may occur in gas analysis through training.

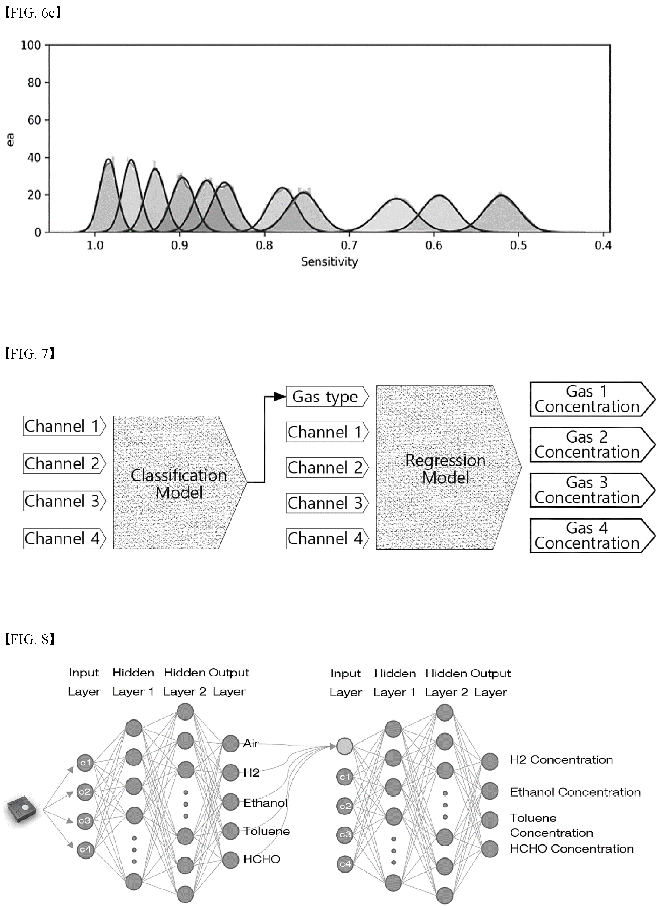

Accordingly, in order to allow distribution data of all sensing sensitivity to be represented, normal distribution data are generated on the basis of an average and a standard deviation of the distribution data of FIG. 6b. This is to generate normal distribution data by normalizing the distribution data as shown in FIG. 6c so that distribution data for all sensing sensitivity are present.

FIG. 6C shows a result of converting distribution data by sensing sensitivity into normal distribution data. The data processing part (106) may set the normal distribution data as training data for a deep learning model and construct a training data DB.

Here, in order to construct a DB with high-quality training data, dispersion among the gas sensors (30, 32), dispersion among the sensing electrodes (170), dispersion due to a measurement error, and the like should be considered, and for this purpose, as many sensing sensitivity values as possible are required.

72 sensing sensitivity values presented in the present disclosure are provided merely as an example for convenience of description, and it is more preferable to construct the training data DB on the basis of a larger number of sensing sensitivity values. Preferably, 700,000 or more training data may be constructed. Since it is difficult to acquire sensing sensitivity over all continuous ranges even with 700,000 training data, virtual training data may be generated by normalizing the distribution data on the basis of an average and a standard deviation of the distribution data. Accordingly, accurate gas analysis without error may be enabled even for unlearned data.

FIGS. 6a to 6c illustrate an example in which a training data DB for sensing sensitivity of a first gas detected by the first sensing electrode is constructed. Accordingly, in order to construct a training data DB for second, third, and fourth gases, it is necessary to construct a training data DB for the second, third, and fourth gases through the same process as that of FIGS. 6a to 6c.

FIG. 7 is an exemplary diagram illustrating a deep learning training process according to an embodiment of the present disclosure. According to this diagram, in the present disclosure, a two-stage learning model may be applied to analyze gas.

The learning models may be generated by training a deep learning training algorithm by using pre-prepared training data. The training data may be sensing sensitivity data generated as shown in FIG. 5 and FIGS. 6a to 6c.

A first-stage learning model may be a classification model. A second-stage learning model may be a regression model.

The first-stage classification model may receive sensing sensitivity of a plurality of gases, be trained by using the sensing sensitivity data, and output a type of each gas.

The second-stage regression model may receive sensing sensitivity of a plurality of gases and gas types, be trained by using the sensing sensitivity and gas type data, and output a concentration of each gas.

FIG. 8 is an exemplary diagram illustrating a deep learning model according to an embodiment of the present disclosure. In the present disclosure, the learning model may include the first-stage classification model and the second-stage regression model.

The classification model may be a fully connected neural network (FCN).

The regression model may be multiple linear regression (MLR).

As a training method, supervised learning may be performed such that the FCN predicts a type of each gas and the MLR predicts a concentration of each gas.

In one example of the drawings, the FCN may receive gas sensing sensitivity of four channels (c1, c2, c3, c4). That is, for example, sensing sensitivity of hydrogen, ethanol, toluene, and formaldehyde may be received as an input layer, a hidden layer 1 having 12 nodes and a hidden layer 2 having 24 nodes may be used, and an activation function of the hidden layers may use Rectified Linear Unit (ReLU). In an output layer, Softmax may be used to normalize an output value into a probability value within a range of 0 to 1 so as to perform supervised learning.

The MLR may receive, as an input layer, a gas type output from the FCN and gas sensing sensitivity of the four channels (c1, c2, c3, c4), use hidden layer 1 and hidden layer 2 that are the same as those of the FCN, and also use Rectified Linear Unit (ReLU) as an activation function of the hidden layers. In an output layer, supervised learning may be performed such that a linear concentration value is output without an activation function.

In the present disclosure, in a case of a mixed gas in which different types of gases are mixed, when the gas is determined to be a mixed gas during concentration prediction by the regression model, the regression model may be designed to separately output a concentration for each gas.

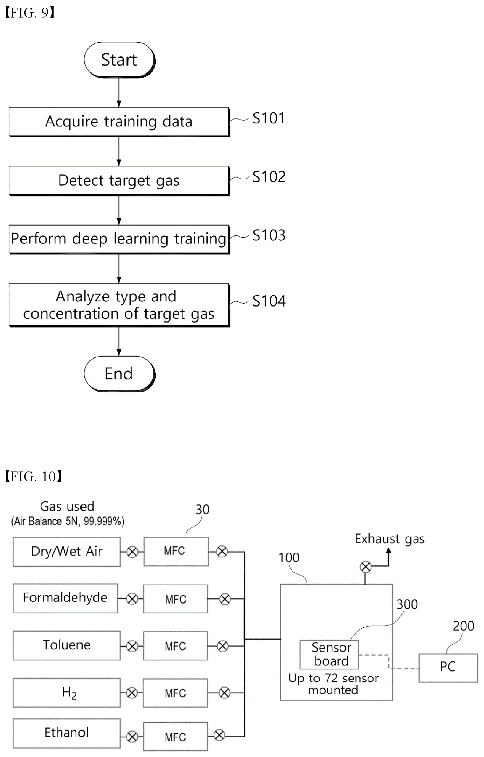

FIG. 9 is a flowchart illustrating a gas analysis method performed in an embodiment of the present disclosure.

Referring to FIG. 9, in the gas analysis method according to the present disclosure, training data for a deep learning model may be acquired with respect to sample gas (S101).

The training data may use normal distribution data generated by detecting sample gas by using a gas sensor for sample gas, converting sensing sensitivity data of the sample gas into distribution data by sensing sensitivity, and normalizing the distribution data on the basis of an average and a standard deviation of the distribution data.

Multiple different types of target gases may be detected by the gas sensors (30, 32) (S102).

Deep learning training may be performed by applying sensing sensitivity of the target gas to the learning model (S103).

In the case of deep learning training, the deep learning model may be trained by using training data for sample gas, and thereafter, when a target gas is detected, the trained deep learning model may be trained by using sensing sensitivity of the target gas.

In the analysis part (300), a type and a concentration of the target gas may be analyzed by using the training result of the deep learning model (S104).

In the present disclosure, the deep learning training may use a first-stage FCN and a second-stage MLR.

The FCN may receive sensing sensitivity of a target gas as an input and output a type of the target gas, and the MLR may receive the type of the target gas output from the FCN and the sensing sensitivity of the target gas and may output a concentration of the target gas.

In the present disclosure, the target gas may be ethanol, hydrogen, toluene, and formaldehyde.

FIG. 10 illustrates an experimental process for gas analysis according to the present disclosure.

In this experiment, four types of gases (ethanol, hydrogen, toluene, and formaldehyde) and air were supplied into the internal space of the gas chamber (101). The gas sensors (30, 32) were disposed in the gas chamber (101).

Supply amounts of the respective gases were finely controlled by a mass flow controller (MFC) (33) and supplied in appropriate amounts corresponding to measured concentrations.

Output values of the gas sensors (30, 32) were checked through a PC (or the processor (20)) connected to the gas sensors (30, 32).

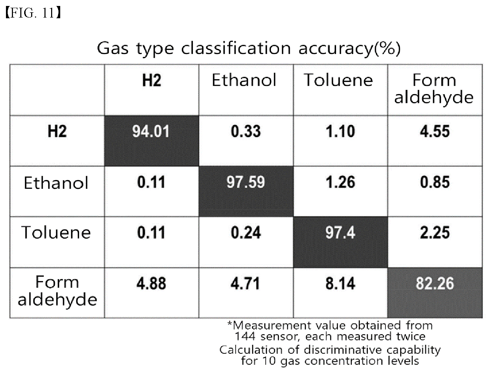

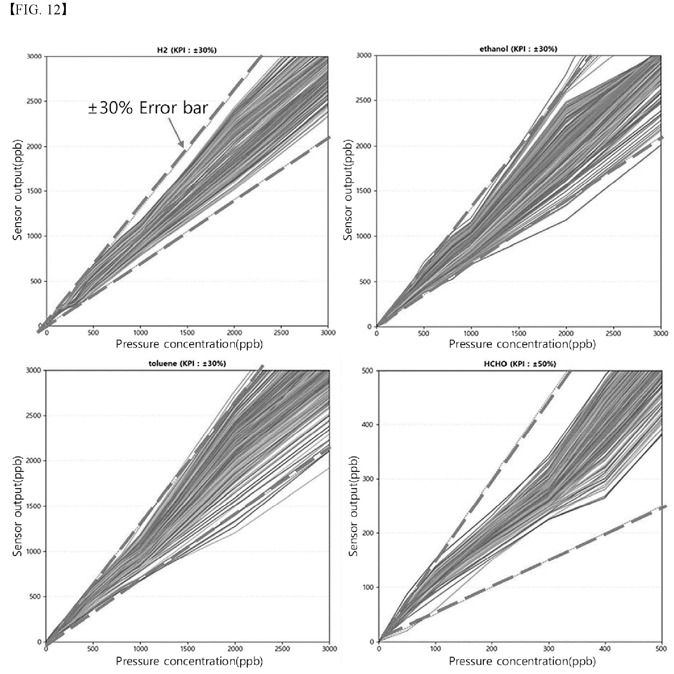

In this experiment, 72 gas sensors were disposed in the gas chamber, and a total of 144 sensing sensitivity values were measured twice. Experimental results are illustrated in FIGS. 11 and 12.

As shown in FIG. 11, discrimination performance (accuracy) of the four types of gases was measured as 94% for hydrogen, 97% for ethanol, 97% for toluene, and 82% for formaldehyde.

As shown in FIG. 12, concentration error rates of the four types of gases were within approximately ±30% for hydrogen, ethanol, and toluene, and within approximately ±50% for formaldehyde.

As described above, the gas sensing device according to the present disclosure may accurately measure harmful gases generated in everyday environments in real time, and thus may be installed in and used together with an air conditioner and an air purifier. Accordingly, by appropriately controlling the air conditioner and the air purifier in association with types and concentrations of detected harmful gases, a comfortable environment may be provided to a user.

As described above, although all the components constituting the embodiment according to the present disclosure have been described as being combined into a single unit or operating in combination, the present disclosure is not necessarily limited to such embodiments. That is, within the scope and spirit of the present disclosure, all the components may operate by being selectively combined into one or more units. In addition, the terms "include," "comprise," or "have," as used herein, unless otherwise specifically stated, imply that the relevant component may be present, and therefore should be construed to include other components rather than to exclude other components.

## Claims

1. A gas sensing device comprising:
a substrate,
a processor installed on the substrate and configured to analyze detected data, and
a first gas sensor configured to detect different types of target gases and a second gas sensor configured to detect different types of target gases, the first gas sensor and the second gas sensor being installed with the processor interposed therebetween,
wherein the first gas sensor is positioned between a first edge of the processor and a corresponding edge of the substrate, and the second gas sensor is positioned between a second edge of the processor and a corresponding edge of the substrate.

2. The gas sensing device of claim 1, wherein the processor analyzes a type and a concentration of each of the detected target gases.

3. The gas sensing device of claim 2, wherein the processor acquires training data for sensing sensitivity of sample gases, trains a deep learning model by using the training data, trains the deep learning model by using sensing sensitivity of the detected target gases, and analyzes a type and a concentration of each of the target gases, wherein the deep learning model comprises a first-stage classification model and a second-stage regression model.

4. The gas sensing device of claim 3, wherein the classification model is a fully connected neural network (FCN), and the regression model is multiple linear regression (MLR).

5. The gas sensing device of claim 4, wherein the FCN receives the sensing sensitivity of the detected target gases and outputs the type of each of the target gases, and the MLR receives the type of each of the target gases output from the FCN and the sensing sensitivity of the detected target gases and outputs the concentration of the target gas.

6. The gas sensing device of claim 1, wherein the processor acquires sensing sensitivity of the plurality of sample gases, generates distribution data of the sensing sensitivity, generates normal distribution data on the basis of an average and a standard deviation of the distribution data, and acquires the normal distribution data as the training data.

7. The gas sensing device of claim 1, wherein one of the first gas sensor and the second gas sensor detects ethanol (C₂H₆O), formaldehyde (HCHO), toluene (C₇H₈), and hydrogen (H₂).

8. The gas sensing device of any one of claims 1 to 7, wherein a temperature/humidity sensor is installed between one edge of the substrate and an edge of the processor.

9. The gas sensing device of claim 8, wherein a through-channel is formed to penetrate the substrate between the temperature/humidity sensor and the processor.

10. The gas sensing device of claim 9, wherein the through-channel is formed to surround the temperature/humidity sensor except for a partial section thereof.

11. The gas sensing device of claim 8, wherein the temperature/humidity sensor, the first gas sensor, and the second gas sensor are respectively disposed around the processor at 90° intervals between corresponding edges of the processor and the substrate.

12. The gas sensing device of claim 1, wherein a connector for signal connection with an external device is installed at one edge of the substrate, wherein the connector is positioned offset toward one side along the edge.

13. The gas sensing device of claim 1, wherein an ESD diode for electrostatic discharge is used on the substrate.

14. The gas sensing device of claim 1, wherein the substrate comprises a cover configured to shield, from an external environment, a surface on which the processor is installed, wherein the cover comprises a processor shielding part formed at a position corresponding to the position of the processor, wherein a first sensor window, a second sensor window, and a third sensor window are respectively formed between corresponding edges of the processor shielding part and the cover.

15. The gas sensing device of claim 14, wherein a partition grid is formed in the third sensor window.
